# DEMANDE DE BREVET EUROPEEN

(11) **EP 3 190 530 A1**
(43) Date de publication de la demande: **12.07.2017**
(21) Numéro de dépôt: 17150211.5
(22) Date de dépôt: 03.01.2017
(51) Int. Cl.: G06F 19/00

(54) **CARTE MÉDICALE DUALE DE GESTION ADMINISTRATIVE ET DE DOSSIER MÉDICAL ET PROCÉDÉS ASSOCIÉS**

(30) Priorité: 07.01.2016 EP 16150402
(71) Demandeur: Coudert, Patrick, 06190 Roquebrune Cap Martin (FR)
(72) Inventeur: Coudert, Patrick, 06190 Roquebrune Cap Martin (FR)
(74) Mandataire: Schuffenecker, Thierry

(57) **Abrégé**

Un support de stockage électronique pour un dossier médical numérique comportant un espace de stockage pour ledit dossier médical numérique, ainsi qu'un programme informatique résidant sur ledit support, ledit support comportant une zone de stockage sécurisé et libre (25) et un port USB (22) ainsi que des premiers circuits (22) configurés pour permettre la création, la consultation et la mise à jour du dossier médical numérique via ledit port USB, dans lequel le support de stockage électronique comporte en outre une puce d'authentification pour le stockage de données administratives afférentes au patient et de second circuits (23, 26) permettant l'authentification du patient via un second canal de communication.

## Description

### Domaine technique de l'invention

La présente invention concerne les systèmes d'information, et notamment une carte médicale duale présentant une double fonction de gestion administrative des droits médicaux et de gestion d'un dossier médical pour la génération, notamment, d'un profil médical d'urgence.

### Etat de la technique

Avec l'essor des moyens de déplacement modernes, la mobilité est à présent au coeur des préoccupations de nos contemporains du 21^{ème} siècle. Avec le besoin qui en résulte de dématérialiser significativement des informations importantes qui le concernent, besoin d'autant plus critique que leur détenteur peut se trouver fort loin de son domicile.

Cette problématique se pose bien évidemment pour les informations médicales concernant tout un chacun, et susceptible de venir constituer un dossier médical propre à son titulaire, auquel ce dernier doit pouvoir avoir accès quel que soit le lieu géographique où il se situe mais également quelles que soient les conditions dans lesquelles cet accès est sollicité.

Les déposants de la présente demande de brevet ont développé différentes techniques visant à développer le concept de Dossier Médical numérique - qu'il soit anonyme ou non - permettant à un citoyen/patient potentiel d'un service médical public ou privé d'utiliser pleinement un dossier médical numérique enrichi de données médicales le concernant.

La demande de brevet n° 08368018.1 du 19 Septembre 2008 (Publication EP2166484), intitulée « *Procédé d'accès à des données nominatives, tel qu'un dossier médical personnalisé, à partir d'un agent local de génération* » décrit une première technique visant à dématérialiser le dossier médical d'un patient venant consulter un groupe de thérapeutes grâce à des procédures spécifiques mises en oeuvre pour assurer un stockage anonyme sur un serveur dit DMA (Dossier Médical Anonyme), lequel stockage sert, lors d'une consultation du patient chez un praticien, à construire, au sein même du cabinet du praticien, une instance du Dossier Médical Nominatif - ou personnalisé - du patient et ce en préservant le caractère confidentiel des informations hautement sensibles que comporte ce dossier médical. De cette manière, il est permis au patient d'aller consulter divers praticiens, notamment au sein d'un même groupement professionnel mais situés en des lieux physiquement éloignés et qui pourront accéder partout au dossier médical personnalisé sans risque que les prestataires informatiques intervenant dans la transmission de l'information puissent briser la chaîne du secret.

La demande de brevet français 11/02726 en date du 8 Septembre 2011 (Publication FR2980019), intitulée *"Procédé d'accès et de partage d'un dossier informatique enrichi par des ressources multimédias personnalisées"* décrit un perfectionnement permettant au patient d'accéder très librement à son dossier médical personnalisé, enrichi par de nombreuses références à des ressources judicieusement choisies par le praticien.

La demande de brevet français 12/00907 en date du 27 Mars 2012 (Publication FR2980020), intitulée *"Procédé d'accès et de partage d'un dossier médical"* décrit un perfectionnement permettant de faciliter le portage du dossier médical entre plusieurs praticiens appartenant ou pas à un même groupement professionnels, voire à un même pays.

La demande de brevet français 12/02401 en date du 10 Septembre 2012 (Publication FR2995431), intitulée « *Procédé d'accès et de partage d'un dossier médical* » décrit un procédé permettant une consultation à distance entre un patient et son praticien, tout en autorisant un accès sécurisé à un dossier médical partagé, nominatif ou non, hébergé sur un serveur tiers. La consultation menée à distance entre le patient et son praticien permet la validation par le médecin de nouvelles données médicales en vue de leur stockage sur le dossier médical partagé et, par suite, la mise à jour de ce dernier.

La demande de brevet français n° 13/01457 déposée le 21 Juin 2013 (Publication FR2999758) décrit un procédé permettant à l'usager de créer un Profil Médical d'Urgence (PMU), i.e. un profil médical public qui est accessible instantanément aux personnels d'urgence tout en conservant la confidentialité du dossier médical dont l'accessibilité est restreinte par des procédures sécurisées. Le PMU est matérialisé sur une application embarquée sur un support de stockage de type USB qui stocke les informations médicales en les sécurisant et en gérant la confidentialité. Ce procédé permet, par ailleurs, dans le même temps, de générer une synthèse automatique des informations médicales enregistrées pour une lecture attractive et efficace auprès des praticiens urgentistes.

Ces demandes de brevets apportent déjà des améliorations significatives au problème du développement d'un véritable dossier médical numérique et susceptible de servir d'assise à une politique moderne de Santé Publique.

Pour autant il demeure souhaitable de pouvoir offrir une solution globale permettant à chaque patient de devenir propriétaire de ses propres données médicales avec un niveau de sécurité et de confidentialité élevé, tout en permettant une gestion fluide de ces mêmes informations confidentielles au sein du cabinet du praticien.

C'est l'objet de la présente demande de brevet.

### Exposé de l'invention

La présente invention a pour objet une solution permettant le stockage d'un dossier médical personnalisé, comportant des données nominatives et médicales, sur une carte médicale offrant un niveau de protection et de confidentialité élevé tout en assurant une exploitation aisée de ces mêmes données lorsque le patient est en consultation au sein d'un cabinet de praticien.

Elle a aussi pour objet de fournir un procédé permettant l'accès à un dossier médical d'urgence d'un grand intérêt pour les services des praticiens urgentistes dès lors qu'un patient inanimé doit être pris en charge.

C'est un troisième objet de la présente invention que de réaliser une carte médicale duale permettant un accès sécurisé, par exemple via un système RFID, aux données administratives du titulaire de la carte médicale, doublé d'une possibilité d'accéder aux données médicales via un port USB.

La présente invention a également pour objet de permettre aux patients de bénéficier de nouveaux services et prestations en relation avec leur dossier médical tout en assurant une grande confidentialité aux données médicales stockées en ce même dossier.

La présente invention a encore pour objet de réaliser un procédé d'accès à un dossier médical nominatif et confidentiel qui puisse être utilisé dans une grande variété de situations, et tout spécifiquement dans les situations d'urgence dans lesquelles un patient se trouve dans l'incapacité d'accéder lui-même à son dossier.

La présente invention a également pour objet de permettre à tout un chacun de constituer un Profil Médical destiné à une situation d'urgence, ou à mettre en avant des problèmes de santé particulier non spécifiquement liés à l'urgence, grâce à une validation médicale de plusieurs niveaux (questionnaire médicalement pertinent, pièces jointes médicales, validation secondaire du praticien...).

La présente invention a ensuite pour objet d'offrir à tout un chacun la possibilité de publier ou de rendre confidentielle chacune des informations médicales auto-renseignées.

La présente invention a pour objet de réaliser un procédé d'accès à un dossier médical nominatif permettant de renforcer l'efficacité des services d'urgences appelés à prendre en charge les patients.

C'est un autre objet de la présente invention que de réaliser une nouvelle carte médicale susceptible de stocker un dossier médical d'urgence non nominatif et non confidentiel, et permettant un accès ultérieur à des données plus sensibles au moyen d'un procédé sécurisé.

L'invention réalise ces buts au moyen d'une carte médicale offrant une double fonctionnalité d'accès comportant un espace de stockage pour ledit dossier médical numérique, ainsi qu'un programme informatique résidant sur ledit support. Le support comporte une zone de stockage sécurisé et libre et un port USB ainsi que des premiers circuits configurés pour permettre la création, la consultation et la mise à jour du dossier médical numérique via ledit port USB.

Le support de stockage électronique comporte en outre une puce d'authentification pour le stockage de données administratives afférentes au patient et des seconds circuits permettant l'authentification du patient via un second canal de communication, qui pourra être RFID par exemple.

De préférence, le support se présente sous la forme d'une carte médicale et dans lequel lesdits second circuits comporte un système RFID permettant une authentification forte, via une communication sans fil, du patient. Alternativement, on pourra prévoir une authentification par carte à puce.

Dans un mode de réalisation particulier, la carte médicale permet la génération d'un Profil Médical d'Urgence généré à partir des informations collectées au sein dudit dossier médical, dans lequel ledit Profil Médical d'urgence est accessible librement via le port USB.

De préférence, la carte médicale comporte des circuits électroniques qui sont configurés pour autoriser un libre accès au dossier médical complet sur double authentification du patient et du praticien, dans lequel l'authentification du patient est réalisé par la mise en oeuvre d'un programme se déroulant chez un praticien et en présence d'une carte professionnelle dudit praticien et dans lequel l'authentification du praticien est établie par la présence de la carte professionnelle dudit praticien et de la présentation de son mot de passe.

De préférence, les circuits électronique sont configurés pour autoriser un accès automatique audit dossier médical sur présence d'un logiciel de facturation ayant également authentifié le praticien.

Dans un mode de réalisation particulier, la carte médicale comporte un programme informatique qui est configuré pour permettre la saisie par le patient d'un dossier Questionnaire médical conçu de manière à permettre la collecte de ses données médicales personnelles en vue de leur stockage au sein d'une base de données disposée au sein de la carte (dans une zone sécurisée). La saisie est associée à l'attribution d'un caractère confidentiel ou non à chacune des informations saisies par le titulaire du dossier médical. Le programme informatique est configuré pour générer automatiquement un profil médical personnel d'urgence correspondant au patient, par exemple dans un format électronique pouvant être disponible en plusieurs langues, ne comportant que les seules données auxquelles ont été attribuées un caractère non confidentiel.

De préférence, le programme informatique est configuré pour permettre la saisie d'une étiquette (Tag Conseil) déterminant un intérêt particulier pour le patient pour chaque élément du Questionnaire médical, de manière à générer un second profil dit « conseil » qui pourra être transmis à un serveur externe de manière à proposer de nouveaux services supplémentaires au titulaire de la carte.

Enfin, l'invention permet la réalisation d'un procédé de génération d'un dossier médical numérique destiné à être stocké sur un support amovible, comportant les étapes :
- branchement dudit support par le port USB à un ordinateur ;
- création d'un nouveau compte et activation de la carte médicale auprès d'un serveur externe ;
- démarrage du programme informatique permettant la saisie par le patient d'un dossier Questionnaire médical conçu de manière à permettre la collecte de ses données médicales personnelles, et le stockage de celles-ci dans une base de données disposée au sein dudit support de stockage, la saisie étant associée à l'attribution d'un caractère confidentiel ou non à chacune des informations saisies par le titulaire du dossier médical ainsi qu'à une étiquette (Tag Conseil) déterminant un intérêt particulier pour le patient ;
- génération automatique d'un profil médical personnel d'urgence correspondant au patient ne comportant que les seules données auxquelles ont été attribuées un caractère non confidentiel ;
- génération d'un second profil médical « conseil » à partir des étiquettes saisies par le patient et transmission dudit second profil « conseil » audit serveur externe.

L'invention permet également la réalisation d'un procédé d'accès à un dossier médical numérique stocké sur un support de stockage, comportant les étapes :
- insertion dudit support dans l'ordinateur d'un praticien consulté par le patient titulaire dudit support ;
- mise en oeuvre d'un code informatique stocké sur ledit support et lecture des données administratives contenues dans ledit support pour procéder à une première authentification d'un patient titulaire dudit support de stockage ;
- lecture d'une carte médicale professionnelle dudit praticien et vérification du mot de passe associé en vue d'une seconde authentification portant sur ledit praticien ;
- en réponse à ladite première et seconde authentification, ouverture du dossier médical numérique pour consultation et/ou mise à jour.

De préférence, le support de stockage est une carte médicale configurée pour faire apparaître la photographie de son titulaire et en ce que lesdits premiers circuits contenus dans ledit support sont configurés pour permettre l'ouverture du dossier médical numérique uniquement à la suite de la vérification par le praticien de la concordance de la photographie avec ledit patient.

En particulier, les premiers circuits de la carte médicale sont configurés pour permettre l'ouverture du dossier médical numérique uniquement à la suite de la vérification que le praticien est bien reconnu par le logiciel de facturation fonctionnant sur ledit ordinateur.

De préférence, le procédé complète l'ouverture du dossier médical numérique (507) pour consultation et/ou mise à jour par l'édition d'une feuille de soins pouvant être directement transmise à un organisme payeur.

### Description des dessins

D'autres caractéristiques, but et avantages de l'invention apparaîtront à la lecture de la description et des dessins ci-après, donnés uniquement à titre d'exemples non limitatifs. Sur les dessins annexés :
La figure 1 illustre l'architecture générale d'un système d'information médicale pouvant servir à l'utilisation de la carte médicale conforme à la présente invention.
La figure 2 illustre l'architecture générale de la carte médicale duale permettant la gestion administrative des droits médicaux ainsi que la gestion du dossier médical et notamment du Profil Médical d'Urgence.
Les figures 3a, 3b et 3c illustrent deux représentations possibles de la carte médicale d'urgence.
La figure 4 illustre un procédé de création de compte et de constitution du dossier médical stocké sur un serveur distant sécurisé dans un premier mode de réalisation de l'invention.
La figure 5 illustre les interaction entre les différentes étapes impliquées dans une consultation basée sur l'utilisation de la carte médicale.
La figure 6 illustre le diagramme d'activité, suivant le formalisme UML, décrivant sous forme de flux (enchaînement d'activités) les étapes de procédé et le comportant du système ou de ses composants.
La figure 7 illustre le diagramme de déploiement , au formalisme UML, représentant les éléments matériels et la manière dont les composants du système sont répartis sur ces mêmes éléments et interagissent entre eux.

### Description des modes de réalisation préférés

L'on décrit à présent comment l'on peut avantageusement combiner, au sein d'un unique dispositif de stockage se présentant, par exemple, sous la forme d'une carte médicale au format « carte de crédit », le stockage d'un dossier médical accessible via un port USB, avec des fonctionnalités d'authentification liées à une puce d'authentification intégrant des données administratives inviolables pouvant servir à la gestion des droits sociaux du titulaire de la carte médicale.

L'on parvient ainsi à combiner les effets liées à la flexibilité du support USB intégrant un logiciel embarqué assurant la gestion nominative du dossier médical et la sécurité liée à une puce réputée inviolable assurant un système d'authentification et d'identification forte du patient ainsi que du praticien concernant les données administratives et celles destinées au remboursement de prestations médicales.

Grâce à une interaction avantageuse entre les deux systèmes qui sont ainsi disposés, comme on va le voir, sur un même support, l'on parvient ainsi à sécuriser l'identification Médecin/patient et également à déclencher un accès automatique au dossier médiale dès lors qu'est établie la reconnaissance d'une « authentification médecin » ou autre thérapeute agrée par le système.

La solution proposée va par ailleurs permettre une évolution vers de nouveaux services fournis par des serveurs externes, et en particulier, la mise en place d'un abonnement selon les services proposées (nouveaux questionnaires, extension à des spécialités paramédicales ou sportives...) ou encore la restitution d'un accès en cas de carte perdue.

Enfin, et c'est non le moindre, la solution proposée permet la création d'un espace personnel public du patient sur un serveur hébergé destiné à un partage d'informations avec des objets connectés, ou au recueil de conseils ou de vidéos personnalisés, avec la conservation d'un espace personnel et confidentiel non hébergé. Le processus a pour objectif de permettre un lien médical anonyme par un « marquage médical » spécifique liée au questionnaire médical, tout en conservant la séparations les informations médicales nominatives et confidentielles qui restent en local sur le support USB afin de conserver l'aspect propriétaire et sécuritaire de ces informations.

### 1. Architecture générale du système d'information

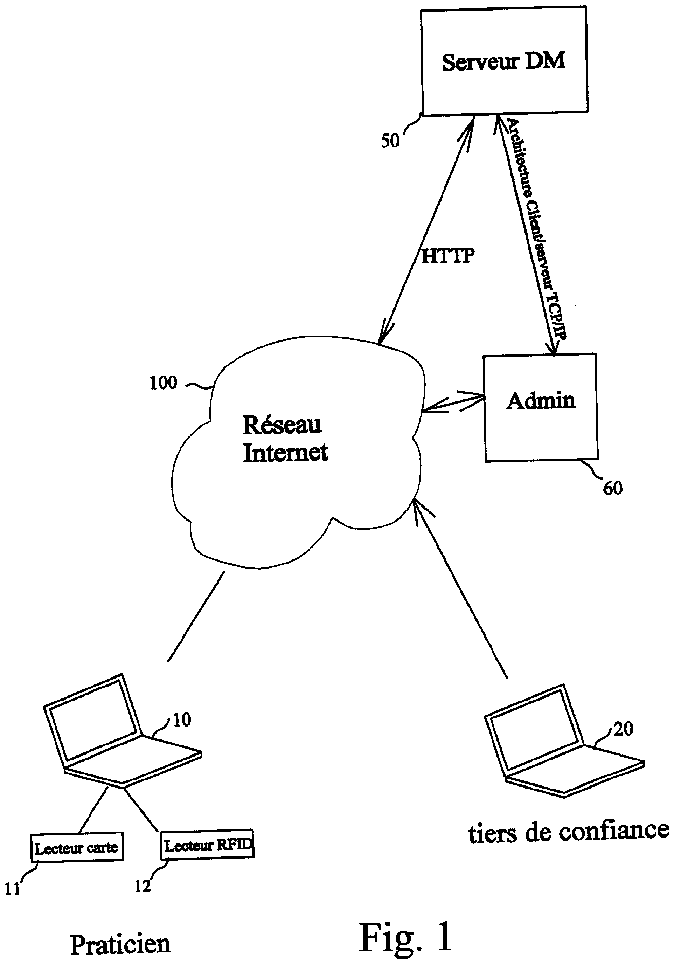

Le schéma de la figure 1 illustre l'architecture générale permettant l'utilisation d'une carte médicale à double fonctionnalité administrative et médicale, où l'on voit un système de traitement de données, représenté sur la figure par un ordinateur portable 10, qui pourra être l'ordinateur d'un praticien ou d'un cabinet médical, disposant d'un accès au réseau Internet 100 et par son biais à un serveur sécurisé distant DM 50, tel que défini notamment dans la demande de brevet PCT/EP2013/003772 en date du 13 Décembre 2015 dont le contenu est intégré dans la présente demande de brevet par référence.

L'ordinateur 10 du praticien se compose de tout ordinateur doté d'un système d'exploitation et d'un ensemble de programmes applicatifs ainsi que des fonctionnalités de communication. En particulier , l'ordinateur 10 sera doté d'au moins un port USB permettant un premier accès aux données figurant sur la carte médicale décrite ci-après.

L'ordinateur 10 comporte également un premier lecteur de carte 11, par exemple un lecteur de carte à puce, qui pourra servir à des fins diverses, et notamment par exemple pour le branchement d'une carte d'authentification permettant d'assurer l'authentification des requêtes émisses par le système 10. L'on pourra par exemple envisager un lecteur destiné à recevoir la **Carte de Professionnel de Santé (CPS)** qui est en usage en France, ou tout système d'authentification forte basée sur l'usage d'une carte à puce... Alternativement, l'on pourra envisager pour le praticien un système d'authentification basée sur une clé USB propre au praticien, voire un système autonome, comme un téléphone mobile intelligent (*smartphone* suivant la terminologie anglo-saxonne) doté de moyens de moyens d'authentification, telle que signature électronique, clés de cryptage.

En outre, l'ordinateur 10 comporte un second lecteur RFID *(Radio Frequency Identification*) 12 permettant d'offrir un second canal de communication distinct aux données présentes sur la carte médicale décrite ci-après, combinant de nouvelles fonctionnalités aux fonctionnalités du port USB, et dont on verra l'intérêt plus loin.

L'on a représenté sur la figure 1, à des fins d'illustration, un serveur administrateur 60 optionnel chargé de l'administration et de la gestion des dossiers médicaux, notamment des inscriptions et de la facturation et, également, de services supplémentaires tels que décrits ci-après.

Un ordinateur 20 détenu par un tiers de confiance pourra, optionnellement, compléter l'architecture pour permettre un accès indirect au dossier médical, tel que définis notamment dans l'enseignement de la demande de brevet PCT/EP2013/003772 déposée le 13 Décembre 2013.

L'on supposera en outre que les systèmes 10 et 20 sont dotés de moyens de notifications, de type courrier électronique, ainsi que d'un navigateur, comme Internet Explorer de l'éditeur MICROSOFT Corp. par exemple, permettant d'accéder simplement, via le protocole standardisé H.T.T.P. (*Hyper Text Transfer Protocol*) au serveur DM 50. De tels moyens sont bien connus d'un homme du métier et ne requièrent pas de développement supplémentaire.

### 2. Structure de la carte médicale à double fonction

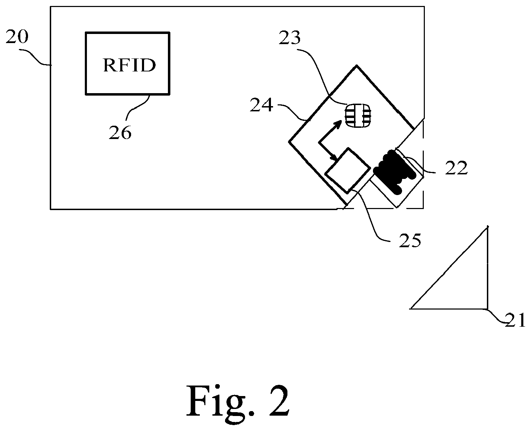

L'on décrit à présent un mode de réalisation particulier d'une carte médicale à fonction duale administrative et médicale, basée sur l'utilisation d'un support physique sophistiqué, représentée dans la figure 2 par la référence 20, pouvant servir aussi bien à la gestion administrative des droits qu'au stockage d'un dossier médical et notamment du Profil Médical d'Urgence (PMU).

La carte pourra même servir opportunément au stockage d'autres types de Profils Médicaux, tel que définis dans la demande de brevet PCT/EP2013/003772 déposée le 13 Décembre 2013, à savoir :
- un Profil Médical Handicap , destiné au personnes souffrant d'un handicap ;
- un Profil Médical Sport pour les sportifs ;
- un Profil Médical Chronique pour les sujets atteints de maladies chroniques ;
- un Profil Médical « baby », enfants, *seniors....* pour les enfants etc...

Par ailleurs, même si le dossier médical a vocation à être stocké sur la carte médicale 20, les circuits électroniques présents sur cette dernière pourront fort opportunément servir, le cas échéant, à des procédures de synchronisation avec le serveur sécurisé 50 ou le serveur 60 gérant les abonnements et notamment les services spécifiques offerts aux patients.

De préférence, comme cela est illustré dans la figure 2, la carte médicale se présente sous la forme d'une carte à puce 20 (dite *SMARTCARD),* ayant un facteur de forme format ID-1 de dimension 85,60 x 53,98 mm, et comportant une puce 23 incorporée suivant la normalisation ISO7816 intégrant des données d'identification sécurisées. Par ailleurs la carte médicale comporte un port USB 22 pour la communication série avec l'ordinateur 10 du praticien, réalisant ainsi un premier canal de communication entre ce dernier et la carte médicale. Celle-ci comporte en outre des moyens de stockage 25 destinés au stockage d'un code d'exécutable pour la mise en oeuvre des fonctionnalités décrites ci-après, et notamment la création et la gestion du dossier médical sécurisé ainsi que les communications avec les serveurs distants 50 et 60. Les moyens de stockage 25 permettent de définir une zone de stockage sécurisée, destinée à recevoir les données nominatives et confidentielles du dossier médical ainsi qu'une zone de stockage libre d'accès, destinée à recevoir le Profil médical d'Urgence. D'une manière générale, les circuits électroniques actifs de la carte 20 pourront être disposés au sein d'une surface 24 intégrant les différents composants électroniques, dont la puce 23 noyés dans le matériau constitutif de la carte. Un capuchon 21 situé dans un angle de la carte 20 permet de recouvrir le port USB lorsque celui-ci n'est pas utilisé.

Comme on le voit, la carte médicale présente un connecteur USB permettant une connexion directe, sans nécessiter de lecteur, à un port USB du système 10. Ce système présente l'avantage de réaliser un premier canal de communication avec la carte qui correspond au mode de connexion le plus répandu.

Par ailleurs, la carte 20 comporte un second canal de communication avec l'ordinateur 10 du praticien, qui pourra être par exemple basé sur un système RFID 26 (**R**adio **F**réquence **Id**entification) composé d'une puce RFID associée à une antenne RFID permettant une communication sans fil, entre la carte médicale 20 et le lecteur RFID 12 de la figure 1, de toutes les données administratives stockées au sein de la puce 23 et utiles pour la gestion des droits, et notamment la vérification que les droits du patient sont encore ouverts ou non. De telles données sont similaires à celles que l'on connaît dans la carte française VITALE et qui permet la mise à jour des droits sociaux du titulaire de la carte médicale. Alternativement, la carte pourra être dotée d'un système NFC (*Near Field Communication*) ou tout système similaire (RFID-HF, RFC...), voire même d'un accès direct à la puce via les contacts électriques conventionnels.

Cette disposition avantageuse, au sein d'un même support physique, d'une fonctionnalité RFID pour la transmission des données administratives de la puce 23 réputées inviolables et de la fonctionnalité USB pour un accès flexible aux données médicales stockées dans la zone de mémoire 25 permet de multiples nouvelles possibilités. Elle assure à la fois la sécurité et l'inviolabilité des données contenues dans la puce 23 et susceptibles d'être lues via le système RFID, tout en permettant également l'enregistrement, la consultation et la mise à jour via le port USB des très nombreuses données - incluant les pièces jointes - constitutives du dossier médical du titulaire de la carte.

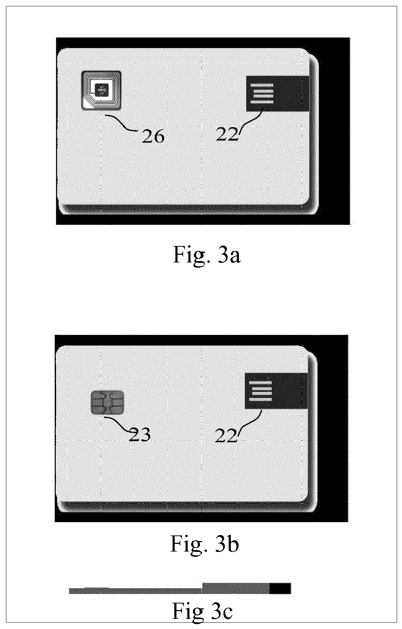

Les figure 3a et 3b illustrent deux représentations possibles de la carte médicale d'urgence, avec une première variante (fig 3a) montrant une carte médicale dotée, à gauche, de son étiquette RFID 26 et, à droite , de son port USB 22.

Lorsque l'on utilise pas la fonction RFID , le transfert des données administratives se fait directement par contact électrique avec la puce 23, comme ce que l'on voit dans la seconde variante de la figure 3b montrant, à gauche, la puce 23 .

La figure 3c montre une vue de profil de la carte médicale où l'on voit que celle ci est susceptible de présenter une double épaisseur pour permettre le logement, d'une part, du système RFID (épaisseur étroite) et, d'autre part, le port USB (épaisseur forte). On observe alors que l'introduction d'une telle carte dotée d'une épaisseur uniforme n'est pas aisée dans un lecteur de carte à puce conventionnel , ce qui pourrait constituer un inconvénient coûteux.

Dans un mode de réalisation, on évite cet inconvénient avec le mode de réalisation de la figure 3a basée sur le système RFID 26 qui permet alors d'accéder très simplement, et sans recours à un lecteur de carte additionnel, aux données administratives contenues dans la puce 23.

Il s'agit de loin de la réalisation la moins coûteuse pour assurer à la fois la sécurité et la performance, en combinant l'inviolabilité de la carte à puce avec l'ultra flexibilité de la communication série que permet le port USB.

On constate donc le grand avantage résidant dans la combinaison d'un système RFID pour la communication des données administratives, notamment de gestion des droits, et du port USB permettant la constitution, la consultation et la mise à jour du dossier médical du patient via l'ordinateur du praticien 10, comme ce que l'on va voir à présent.

### 3. La création du dossier médical et la génération du Profil Médical d'Urgence (PMU)

La carte médicale duale est configurée pour permettre le stockage, au sein de la zone mémoire 25 d'un dossier médical numérique sous une forme quelconque et, plus spécifiquement, dans les modes de réalisation qui seront exposés ci-après, un ensemble de données médicales rassemblées notamment au moyen d'un Questionnaire Médical, spécialement conçu pour collecter de manière cohérente et classifiée diverses données médicales d'un utilisateur/patient potentiel.

Suivant un mode de réalisation, ce dossier médical comporte des données nominatives et confidentielles stockées de manière sécurisée et privative au sein de la carte médicale et implique également la génération préalable - et automatique - d'un dossier médical d'urgence - ci-après désigné Profil Médical d'Urgence (PMU) - lequel est généré à partir des informations saisies par le patient lors de la constitution de son dossier médical.

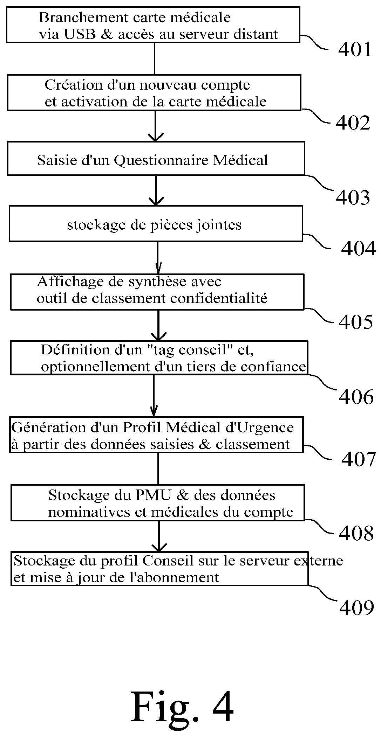

La figure 4 illustre un procédé de création de compte et de constitution du dossier médical numérique - mais également le Profil Médical d'Urgence (PMU) destiné à être stocké dans la zone mémoire de la carte médicale duale

Le procédé démarre par une étape 401 lorsque l'utilisateur branche sa carte médicale 20 au sein d'un port USB de son ordinateur. Alternativement, Il en résulte la reconnaissance de la carte médicale par le système d'exploitation de l'ordinateur et le démarrage d'un code exécutable permettant le déroulement des étapes de procédé décrites ci-après et notamment la transmission d'une requête destinée à un serveur externe, tel que le serveur DM 50 ou le serveur *Admin* 60 de la figure 1.

La requête est reçue par le serveur distant dans une étape 402, entraînant la création d'un nouveau compte auprès de ce même serveur. En effet, la constitution de ce dossier médical suppose l'ouverture d'un compte utilisateur permettant un enregistrement administratif du compte auprès du serveur comportant notamment la génération d'un identifiant et d'un mot de passe qui pourront être librement utilisés par le patient d'une manière conventionnelle pour un accès sans restriction à son dossier médical et notamment en vue de son examen et/ou de sa mise à jour. D'une manière générale, l'identifiant sera créé par le serveur sécurisé distant, soit de toute pièce, soit de manière secondaire à la suite de la présentation d'un autre identifiant primaire, tel que celui utilisé notamment pour l'accès aux services bancaires (Carte MasterCard, Diners, American Express - marques déposées par leurs titulaires respectifs).

Pour l'enregistrement du nouveau compte, le serveur génère un premier formulaire ou questionnaire de saisie d'informations d'ordre administratif, et notamment nominatives: tels que, par exemple: nom, date et lieu de naissance, adresse, téléphone, adresse courriel etc ...

Une fois les données administratives saisies, l'étape 402 s'achève par l'activation de la carte médicale qui formalisera le démarrage de l'abonnement de l'utilisateur auprès du service, lequel abonnement pourra également permettre la gestion de services spécifiques, comme ceux qui seront décrits ci-après.

Puis dans une étape 403, le procédé procède, au moyen d'une interface graphique de saisie appropriée, à l'affichage d'un Questionnaire Médical se composant d'un ensemble de formulaires ou sous-questionnaires médicaux soigneusement élaborés et conçus de manière à permettre une collecte d'un grand nombre d'informations ou données médicales concernant le patient. La présentation du questionnaire pourra inclure des zones d'explication des questions, et fournir/présenter automatiquement des conseils adaptés en fonction de la réponse enregistrée.

Ces questionnaires peuvent être remplis soit par le patient lui-même, soit conjointement par le patient et son médecin, notamment lors d'une consultation qui pourra être une consultation dématérialisée telle que décrite dans la demande de brevet français 12/02401 en date du 10 Septembre 2012 intitulée « *Procédé d'accès et de partage d'un dossier médical »,* et déposée par les demandeurs de la présente demande de brevet, qui présente l'avantage de permettre une validation immédiate par un professionnel de la Santé des données médicales ainsi saisies sur le serveur sécurisé. De manière plus générale, l'on pourra envisager l'utilisation des procédures décrites dans les demandes de brevet mentionnées précédemment, notamment pour permettre l'enrichissement du dossier médical stocké sur la carte médicale 20.

Lorsque c'est le patient /utilisateur qui instruit lui-même son dossier médical, le patient remplit lui-même les questionnaires qui lui sont soumis par l'interface du serveur sécurisé, en attendant que celui-ci puisse être validé par un praticien de son choix.
- Il pourra lui-même remplir les informations concernant sa santé en suivant un questionnaire précis, qui pourra s'afficher et s'enregistrer sous plusieurs langues.
- Ce questionnaire contiendra plusieurs catégories de questions destinées à intéresser les services d'urgences, mais il peut aussi contenir des questions plus spécifiques liées à des profils particuliers (sport, maladies chroniques, handicap, autonomie, psychisme, dépendance...)
- Ce questionnaire permettra tout type de question (questions à boulets, boutons radios, choix multiples, questions liées...) dont l'objectif de son organisation sera de formuler toute question d'une manière la plus intelligible et simple possible à son utilisateur afin d'obtenir les réponses les plus médicalement exactes et pertinentes.
- La synthèse de ce questionnaire aura pour objectif de présenter les informations saisies par le patient dans un ordre de priorité utile aux urgentistes (système de pondération qui affichera automatiquement les réponses dans un ordre souhaité par le corps médical, et non dans l'ordre saisi par le patient)

Dans un mode de réalisation particulier, l'interface graphique de saisie du questionnaire comporte un outil permettant le stockage, au sein de la zone de mémoire sécurisée 25 de la carte médicale 20, de pièces jointes, des fichiers électroniques de formats quelconques - notamment JPG, BITMAP, TIFF, PDF etc - comme cela est illustré dans une étape 404 de la figure 4.

Il est à noter que ces pièces jointes représentatives de prescriptions, de certificats, de rapports d'analyse etc... présentent un grand intérêt dans la mesure où elles permettent de venir conforter une validation ultérieurement effectuée par le médecin du patient. Optionnellement, et suivant l'enseignement de la demande de brevet PCT/EP2013/003772 déposée le 13 Décembre 2013, ces pièces jointes constitutives du dossier médical personnalisé pourraient être téléchargées sur le serveur externe DM 50, dans l'hypothèse où le patient donne son accord à un tel stockage hors sa carte médicale.

Lorsque le Questionnaire Médical est complètement rempli par l'utilisateur, le procédé procède ensuite à l'affichage d'un écran ou plusieurs écrans de synthèse, dans une étape 405, venant résumer les différents éléments d'information composant les différents sous-questionnaires constitutif du Questionnaire Médical.

Dans un mode de réalisation, l'interface graphique proposé au titulaire, lors de l'affichage des écrans de synthèse, propose un outil de classification lui permettant d'affecter ou non un caractère confidentiel à chacun des éléments constitutifs du Questionnaire Médical. Cette fonctionnalité est, comme cela a été décrit dans la demande de brevet PCT/EP2013/003772 déposée le 13 Décembre 2013, particulièrement importante puisqu'elle a vocation à confier au patient le libre arbitre en ce qui concerne le caractère confidentiel ou non qu'il convient d'attribuer à chacun des éléments d'information constituant son *propre* Dossier Médical et, par suite, de permettre une génération automatique et circonstanciée du Profil Médical d'Urgence (PMU), qui pourra servir de premier lien entre un praticien, par exemple un médecin urgentiste appelé à officier et, par suite, de vecteur d'accès au dossier médical complet de ce dernier.

Puis, dans une étape 406, l'interface graphique propose au titulaire de la carte un second outil de classification lui permettant d'affecter, à chacun des éléments constitutifs de son Questionnaire médical, une étiquette « conseil » (ou ***Tag Conseil*)** destiné à définir si le titulaire de la carte souhaite bénéficier d'informations circonstanciées (et commerciales) relatives à l'aspect correspondant du Questionnaire. Cette étape de définition du « *tag conseil* » est particulièrement avantageuse puisqu'elle permet au procédé, outre la génération ultérieure du Profil Médical d'Urgence, la définition d'un second profil « Conseil » axé sur des services extérieurs qui seront susceptibles d'être offerts au titulaire via les serveurs externes DM50 et/ou Admin 60, qui auront à gérer l'abonnement du titulaire.

Dans un mode de réalisation, le procédé ne valide le Questionnaire Médical (et par conséquent tous les sous-questionnaires le composant) qu'après l'achèvement complet de la classification opérée par le patient.
- Le patient aura donc décidé de publier ou non chacune des informations contenue dans la synthèse (considération faite de ce que l'article 8 de la Loi Informatique et Liberté permet à toute personne de rendre publiques, les informations qui le concernent) qui s'enregistreront sous la forme d'un document accessible sur le serveur sécurisé et agréé.
- Ce document pourra être enregistré via le port USB de la carte.

Optionnellement, suivant l'enseignement de la demande de brevet PCT/EP2013/003772 déposée le 13 Décembre 2013, le serveur sécurisé DM50 peut solliciter du titulaire du compte nouvellement crée la désignation d'au moins un tiers de confiance, avec la saisie d'éléments d'identification tel que le nom, les coordonnées téléphoniques, l'adresse courriel etc... Dans un mode de réalisation particulier, le titulaire du compte se voit offrir la possibilité de désigner plusieurs tiers de confiance (au minimum 3) qui se verront formellement attribuer une clé de substitution, par exemple sur un support physique telle qu'une carte, laquelle sera également enregistrée dans une table stockée dans la zone protégée du dossier médical du patient permettant un accès indirect au Dossier Médical stocké au sein de la carte médicale duale 20.

Puis, dans une étape 407 - et il s'agit d'un élément particulièrement avantageux - le procédé procède à la génération automatique d'un Profil Médical d'Urgence (PMU) comportant des informations vitales, quoique non nominatives et non confidentielles, et judicieusement présentées dans leur ordre d'importance et de pertinence pour les besoins d'un service d'urgence. De préférence, le PMU sera multilinguistique et les circuits électroniques de la carte médicale seront configurés pour reconnaître automatiquement le pays d'accueil de l'ordinateur accédant à la carte et afficher le PMU dans la langue d'accueil déterminée. Clairement, il ne s'agit que d'exemples non limitatifs. Ce Profil Médical d'Urgence (PMU) est généré automatiquement en fonction des informations collectées lors du remplissage des formulaires (et ultérieurement validées par le médecin), mais également en fonction des diverses classifications opérées par le titulaire du compte qui, au cas par cas, aura attribué un attribut de confidentialité à chacune des données saisies lors de l'étape 405.

On rappellera que le Profil Médical d'Urgence (PMU) se distingue fondamentalement du dossier médical numérique, tant dans sa structure interne que dans sa fonction.

D'abord, s'agissant de sa structure, le Profil Médical d'Urgence (PMU) est un ensemble de données, généré automatiquement suivant le procédé de la figure 4, et qui ne comporte que des informations **NON NOMINATIVES** et classifiées **NON CONFIDENTIELLES,** ainsi qu'une évocation - non confidentielle - d'informations confidentielles susceptibles de se trouver dans le dossier médical stockées dans le serveur sécurisé. Par conséquent, si ce dernier est voué à s'enrichir continuellement, au rythme des aléas et des interventions qui ponctuent la vie du patient, le Profil Médical d'Urgence (PMU) a - lui - une vocation à une certaine stabilité puisqu'il constitue un résumé, un abstract, et recense les données les plus importantes - non nominatives et non confidentielles - pour un service d'urgence susceptible de prendre un jour en charge le patient.

Par ailleurs, s'agissant de sa fonction, l'on va constater, avec les procédures qui sont décrites ci-après, que le Profil Médical d'Urgence (PMU) sert de clé, de vecteur d'accès au dossier médical dans le cadre d'une procédure d'urgence. A cet effet, le PMU permet un accès direct au serveur sécurisé dans le but de permettre l'ouverture complète du dossier médical du patient, notamment via le recours à l'un des tiers de confiance préalablement désignés par le titulaire du dossier médical. Dans un mode de réalisation particulier, le Profil Médical d'Urgence (PMU) comporte un identifiant ou un lien d'accès non nominatif vers le ou les tiers de confiance désigné(s) par le titulaire.

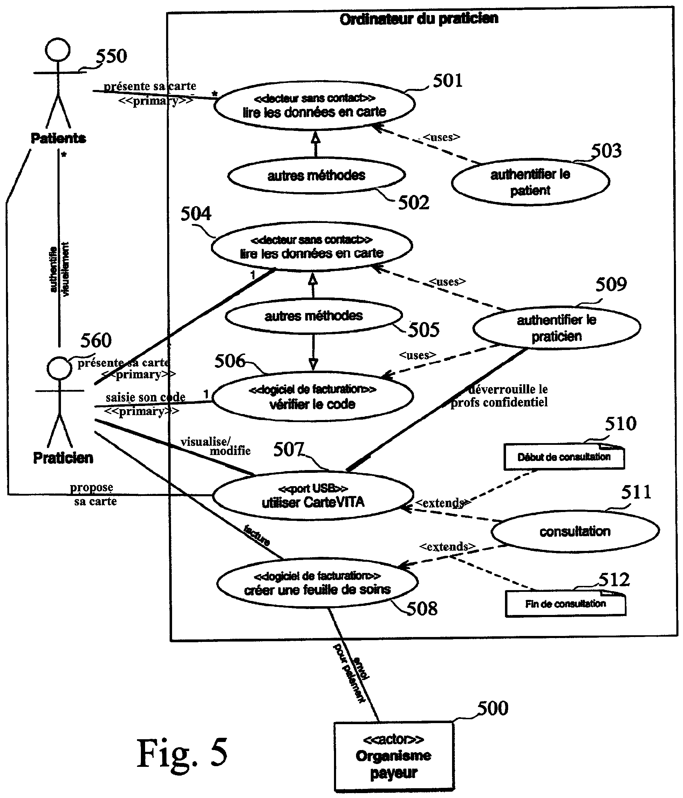

En revenant à nouveau au procédé de la figure 4, l'on voit que, à la suite de la génération du Profil Médical d'Urgence (PMU) de l'étape 407, le procédé procède, dans une étape 408, au stockage de ce dernier au sein d'une zone de mémoire accessible librement au sein de la carte médicale 20, contrairement aux aux autres informations nominatives et/ou confidentielles qui sont stockées dans une zone sécurisée au sein de la même carte médicale et qui ne seront accessibles, pour consultation, qu'au moyen de la présentation de l'identifiant du titulaire du compte **avec** son mot de passe associé ou, alternativement, avec une clé de substitution suivant les procédures d'accès d'urgence telles que décrites dans la demande de brevet PCT/EP2013/003772 déposée le 13 Décembre 2013 ou, plus avantageusement encore, avec le procédé décrit dans la figure 5 ci-après.

Au terme de la procédure de création de compte et d'enregistrement du Questionnaire Médical, le procédé transmet, dans une étape 409 le second profil « conseil » aux serveurs externes, par exemple le serveur DM50 et/ou le serveur Admin 60 afin d'en permettre la gestion , notamment au niveau de l'abonnement.

Il est à noter que le titulaire du compte, détenant l'identifiant celui-ci ainsi que le mot de passe y associé, pourra bien évidemment accéder à nouveau ultérieurement à son compte de manière à mettre à jour son dossier médical en venant y intégrer de nouvelles données et/ou pièces jointes. Il pourra également, modifier la ou les désignations des tiers de confiance en tant que de besoin, et les mises à jour ainsi faites seront clairement répercutées, lorsque cela sera nécessaire (notamment en ce qui concerne la désignation des tiers de confiance) au niveau du Profil Médical d'Urgence.

Comme on le voit, si le Profil Médical d'Urgence est de libre accès à tout un chacun - dès lors que l'on branche à un ordinateur la carte médicale via son port USB - les données nominatives et confidentielles restent particulièrement protégées au sein de la zone mémoire sécurisée de la carte médicale.

Cette protection efficace des données nominatives et confidentielles n'empêche toutefois pas - et cela est un avantage significatif de la présente invention - un accès automatique à ces dernières dès lors que le patient se trouve au cabinet du praticien.

En effet, comme cela ressortait clairement de l'enseignement de la demande de brevet PCT/EP2013/003772 précitée, l'accès aux données nominatives et confidentielles du dossier médical était permis dès lorsque le patient dispose de ses identifiant et mot de passe ou, à défaut, via la clé de substitution obtenue auprès du tiers de confiance désigné dans l'étape 406 de la figure 4 (pouvant prendre des formes diverses : SMS, courriel, appel vocale etc...)

Mais, comme on va le voir à présent, la solution proposée permet un accès automatique même aux données nominatives et médicales stockées dans la carte médicale en réponse à une double authentification du praticien et du patient.

### 4. L'ouverture automatique du dossier médical en réponse à la double authentification du patient/praticien

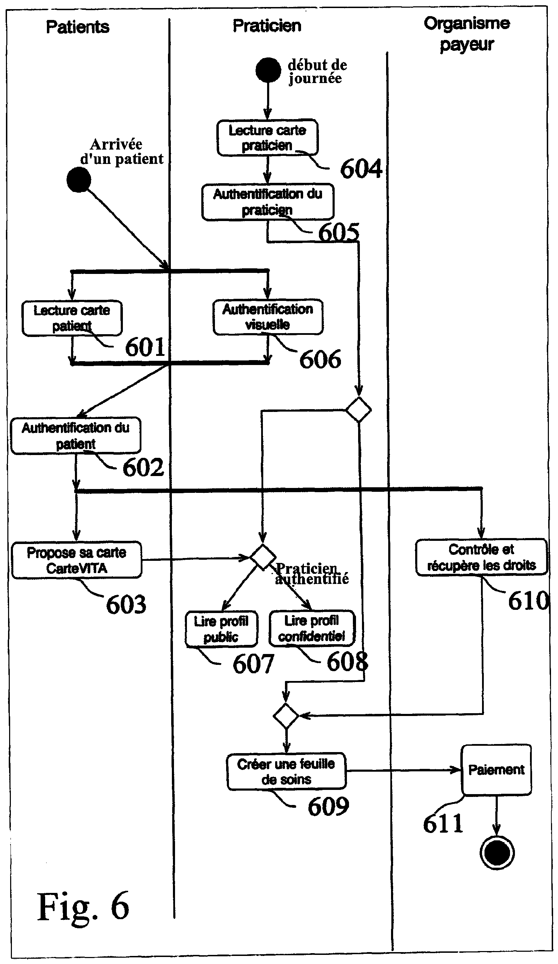

Les figures 5 et 6 illustre un mode de réalisation de l'invention permettant un nouveau mode de certification du patient et/ou du praticien dans le but d'autoriser une ouverture automatique du dossier médical nominatif et confidentiel.

L'on notera que cette nouvelle possibilité d'accès qui est proposée est susceptible de servir une grande variété de situations.

D'abord, cet accès automatique au dossier médical peut naturellement servir les situations d'urgence, comme celles mentionnées dans la demande de brevet PCT/EP2013/003772 déposée le 13 Décembre 2013.

Mais plus généralement, cet accès automatique au dossier médical peut être mis en oeuvre dans toute autre situation de suivi médical par un thérapeute afin de permettre une meilleure coordination des soins.

A cet égard, la carte médical et le logiciel qui y est embarqué est configuré pour vérifier l'authentification du praticien. En effet, d'une manière générale, le praticien possède une carte contenant une puce sans contact (RFID ou autre). Cette puce contient l'identifiant du praticien, ainsi que quelques données administratives (nom, secteur d'activité, ...). Un logiciel de facturation est installé sur le poste du praticien. Celui-ci permet de lire les données du praticien et du patient stocké sur leurs cartes respectives. Ces données ne sont pas modifiables (par le praticien ou le patient).

De son côté, le patient est titulaire de sa carte médicale telle que décrite en relation avec la figure 2.

L'authentification du patient se fera par le praticien qui pourra vérifier la carte du patient et la présence, sur celle ci, des données imprimées le concernant (nom, prénom, photo, ...).

Inversement, l'authentification du praticien se fera avec la présence de sa carte, ainsi qu'un code qu'il devra saisir sur le logiciel de facturation.

Les droits du patient seront accessibles en ligne, sur le serveur de l'organisme payeur, à partir du logiciel de facturation. Le logiciel de facturation pourra créer une feuille de soins à partir des données provenant des cartes patients et praticiens ainsi que du serveur de l'organisme payeur, et envoyer le résultat à cet organisme payeur.

On constate donc que cette double authentification, tant du praticien que du patient, peut avantageusement servir la mise en oeuvre d'un nouvel accès au dossier médical automatique et, au final, .

Le diagramme de la figure 5, au formalisme UML, illustre cette coopération entre la carte professionnelle du praticien et la carte médicale du patient. On y recense les différentes possibilités d'interaction entre le système (l'ordinateur du praticien) et les acteurs (les patients 550, le praticien 560, l'organisme payeur 500), soit les fonctionnalités

En particulier l'on constate qu'un patient 550 présente sa carte au praticien 560 qui peut alors l'insérer dans son ordinateur de manière à en lire - par liaison RFID par exemple - les données administratives (bloc 501).

Cette lecture, peut être complétée par toute autre méthode (bloc 502) pour assurer, finalement une première authentification du patient (bloc 503). Parmi les autres méthodes possibles, l'on notera que l'on pourra prévoir une authentification visuelle du patient par le praticien qui pourra vérifier la concordance des données administrative (et/.ou la photo éventuellement présente sur la carte médicale) pour confirmer l'authentification du patient.

Le procédé comporte ainsi une première authentification forte du patient, résultant de la mise en oeuvre des programmes stockées sur sa carte médicale.

S'ajoute l'authentification forte du praticien lui-même qui, comme on le voit sur la figure 5, présente sa propre carte professionnelle et l'insère dans le lecteur de carte 11 de son ordinateur qui peut alors y lire les données (bloc 504). Toute autre méthode (bloc 505) peut également venir conforter cette seconde authentification forte (bloc 509) qui résulte de cette procédure. En particulier, en saisissant son code (primary), le logiciel de facturation s'exécutant dans l'ordinateur du praticien peut, lui également, venir conforter cette seconde authentification forte liée au praticien (block 509).

Les programmes exécutables présent aussi bien sur la carte médicale du patient et ceux de l'ordinateur 10 du praticien sont configurés, depuis le début de la consultation (bloc 510) jusqu'à la fin de cette dernière (bloc 511-512) pour exploiter cette double authentification, tant du patient que du praticien, pour permettre un déverrouillage du dossier médical du patient et, par suite, un accès libre aux données nominatives et médicales stockées dans celui-ci.

Au terme de la consultation (bloc 511), le logiciel de facturation fonctionnant au sein de l'ordinateur du praticien est à même de générer une feuille de soins (bloc 508) qui pourra être immédiatement transmise à l'organisme payeur 500 pour traitement.

Comme on le voit sur le diagramme de la figure 5, la carte médicale du patient comporte des circuits électroniques 22-26 qui sont configurés pour vérifier une double authentification du patient et du praticien :
- authentification du patient par la reconnaissance du logiciel tournant dans l'ordinateur du praticien ;
- authentification du praticien par la détection de sa carte professionnelle et/ou la saisie du mot de passe de celui-ci, notamment au sein du logiciel de facturation.

Une fois cette double authentification établie, la carte médicale est configurée pour permettre, au sein de l'ordinateur hôte du praticien, un accès libre au données nominatives et confidentielles composant le dossier médicale du patient.

L'on obtient ainsi un nouveau processus de distribution d'une clé de distribution et d'ouverture du Profil médical confidentiel peut-être proposée :
L'authentification du praticien, combinée à celle du patient, déclenche à la fois le processus de mise en oeuvre du système de facturation qui est en général effectué par la secrétaire du cabinet médical (qui peut posséder une carte équivalente à celle du praticien) mais il déclenche également un droit d'ouverture du profil Médical confidentiel de l'applicatif médical embarqué sur le port USB via un serveur distant qui authentifie le praticien référencé et génère automatiquement une clé de substitution temporaire, selon des mécanismes similaires à l'activation ou à la gestion d'un abonnement, ou ceux décrits dans la demande de brevet précitée.

La figure 6 illustre le diagramme d'activité, suivant le formalisme UML, décrivant sous forme de flux (enchaînement d'activités) les fonctions/étapes de procédé et le comportement du système ou de ses composants en distinguant les différents intervenants : patients - praticien - organisme payeur :

### Pour le patient :

Etape 601 : lecture de la carte du patient au sein de l'ordinateur 10 du praticien
Etape 602 : authentification du patient
Etape 603 : lecture des données administratives (RFID) de la carte médicale

### Pour le praticien :

Etape 604 : lecture de la carte du praticien au sein de l'ordinateur 10
Etape 605 : authentification du praticien
Etape 606 : authentification visuelle (venant compléter l'authentification du patient résultant de la possession de la carte médicale)
Etape 607 : lecture du profil médical d'urgence (en cas de non déverrouillage)
Etape 608 : lecture du dossier médical complet (en cas de déverrouillage)
Etape 609 : génération de la feuille de soin à l'attention de l'Organisme payeur

### Pour l'organisme payeur :

Etape 610: contrôle et récupération des données réprésentatives des droits sociaux
Etape 611 : traitement de la feuille de soins et paiement.

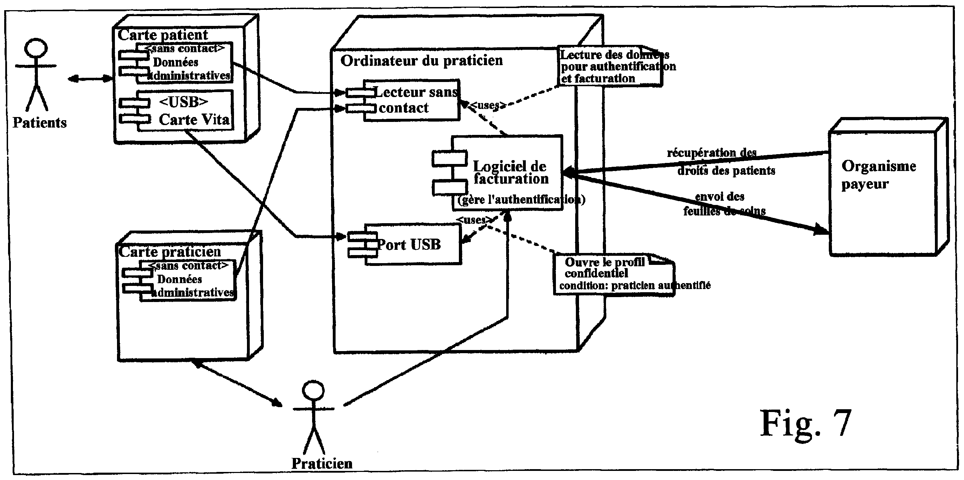

La figure 7 illustre le diagramme de déploiement , au formalisme UML, représentant les éléments matériels et la manière dont les composants du système sont répartis sur ces mêmes éléments et interagissent entre eux :
- la carte médicale du patient selon la présente invention ; .
- la carte du praticien ;
- l'ordinateur du praticien ;
- l'organisme payeur

### 5. Autres mode de réalisation possibles

Les diagrammes qui viennent d'être décrits en détail montrent un système de stockage des données servant à l'authentification ainsi qu'à la facturation sur un support sans contact (comme RFID).

D'autres systèmes peuvent être utilisés, que ce soit pour l'authentification du praticien et des patients, ou pour le stockage des données administratives.
Ces autres systèmes peuvent être:
✔ Une carte à puce, telle que représentée dans la figure 3b
✔ Système biométrique, comme la lecture des empreintes digitales.
✔ Token USB.

### 6. Processus d'activation

En utilisant un applicatif embarqué sur un support externe, et en laissant la totale maîtrise des données médicales à l'utilisateur, il apparaît indispensable pour des raisons de sécurité mais aussi pour permettre une restitution de carte en cas de perte, ou pour assurer un support aux utilisateurs, d'établir un lien internet entre la carte USB et l'utilisateur final.
En utilisant un système de va et vient avec différents serveurs distants, cela permet de dissocier le numéro d'identification de la carte du support physique lui-même (conformité CNIL) et de le créer à un espace personnel hébergé comportant notamment une adresse mail et une identification nominative de l'utilisateur.

Cet espace personnel hébergé liant la carte et son utilisateur offre ainsi de nombreuses possibilités commerciales ou de services annexes tout en conservant l'absolue confidentialité des données médicales qui restent stockées sur la carte et accessible uniquement par le port USB.
Il est donc nécessaire d'activer la carte avant de pouvoir l'utiliser, et une partie de cette activation doit se faire en ligne, sur un serveur distant. Par ce processus d'activation il est alors possible d'identifier chaque client, par son adresse email (moyen de contact ultérieur), plus quelques autres données (sexe, âge, nom et prénom).
Par ce procédé, il est possible de créer pour chaque utilisateur un compte en ligne avec un espace personnel, permettant de faire des achats supplémentaires (abonnement), de télécharger des mises à jour adaptées à son produit...
Les étapes sont les suivantes:
a) Le client saisit son numéro de carte sur le site serveur, numéro qui est délivré par un serveur dédié qui gère par groupe de numéro des catégories éventuelles de population (selon le type de distribution de la carte, la catégorie de patients (handicap, sport,) ou selon d'éventuelles associations ou autres groupes commerciaux...
b) Le numéro de carte permet également de définir des droits pour chaque groupe d'utilisateur (abonnement, durée, catégorie de questionnaires...).
c) Le client saisi des données administratives sur le site serveur (dont son email, et un mot de passe pour son compte en ligne). Ce système permet de gérer entre autre la réinitialisation d'un mot de passe est basé sur la connaissance de l'email lié au numéro de la carte.
d) Un email est envoyé au client, contenant une URL sur lequel il doit cliquer. Cela permet de vérifier que le seul moyen de contact (email) est juste, et éviter la création de comptes anormaux.
e) Le client visualise son code d'activation de la carte. Il peut le copier-coller sur l'écran de la carte, obligeant ainsi l'utilisateur à faire l'activation.
f) Le client saisit un identifiant et mot de passe pour accéder à sa carte, puis peut saisir son profil, augmentant ainsi la sécurité pour la modification du profil. Ce mot de passe devient alors inconnu du serveur octroyant à l'utilisateur une sécurité supplémentaire.

### 7. Abonnement à de nouveaux services commerciaux

Un abonnement est une durée associée à une carte, permettant de saisir et modifier la totalité de son profil. Il ne peut être géré que par un serveur distant qui définit les durée et règles d'accès selon les services liés à l'abonnement (questionnaires spécifiques, alertes médicales...).
Cette gestion de l'abonnement permet par exemple de distinguer au sein du questionnaire des zones de questionnaires médical qui peuvent être liées à un abonnement ou au contraire libre de droit d'accès indéfiniment. C'est le cas d'un questionnaire médical strictement lié à la notion d'urgence qui doit rester libre d'accès à vie.
En revanche des questions plus spécifiques sur des pathologies ciblées et ou spécifiques, de l'activité sportive ou d'autres domaines paramédicaux de nutrition ou de bien être peuvent ainsi bénéficier de la mise en place d'un abonnement lié aux services proposés.

Les étapes de vérification sont les suivantes:
a) Lorsque le client cherche à modifier son profil, le logiciel contacte le serveur de base et demande si l'abonnement associé à cette carte est toujours valide.
b) Si oui, le client peut modifier son profil. Si non, il a une alerte, et ne peut pas modifier son profil.

Les étapes de vérification peuvent se décrire de la façon suivante:
a) Lorsque le client cherche à modifier son profil, le logiciel contacte le serveur de gestion et demande si l'abonnement associé à cette carte est toujours valide.
b) Si oui, le client peut modifier son profil en totalité. Si non, il a une alerte, et peut modifier la partie liée à la zone définit sans abonnement comme les questions d'urgences (par exemple) de son profil lié à l'abonnement qui devient alors est inaccessible.

*NB :* il est possible de proposer d'autres modes d'accès à l'abonnement basées toujours sur le même principe en laissant par exemple la visualisation du profil, contenant toutes les données saisies, sans les lier à un abonnement. Ce procédé permet toutes les combinaisons possibles.

### 8. Ré-initialisation du mot de passe.

La réinitialisation de mot de passe, doit comporter des sécurités que l'on peut décrire de la façon suivante :
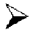 Quelqu'un qui vole la carte ne peut changer le mot de passe. Ce qui exclut les systèmes à base de phrase secrète, ainsi que tout système basé uniquement sur le support de la carte.
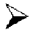 La société destinée à distribuer notre invention ne connaît pas le mot de passe de l'utilisateur, et donc ne peut créer un nouveau mot de passe. Ce qui exclut qu'on puisse le faire manuellement ou automatiquement, ce qui reviendrait à un accès aux données par le personnel de l'entreprise concernée.
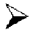 La méthode de réinitialisation de mot de passe doit être unique pour chaque carte. Un client réinitialisant son mot de passe ne doit pouvoir le faire sur une autre carte que la sienne.

Les étapes sont les suivantes:
a) Le client saisit son numéro de carte, et est redirigé sur le site de la société. (On doit connaître le numéro de carte pour ne réinitialiser que celle-ci.)
b) Le site affiche l'information qu'un mail est envoyé à l'utilisateur. (Le mail est associé à ce numéro de carte, et correspond à l'utilisateur qui a activé sa carte. Si quelqu'un a volé la carte, il ne recevra jamais ce mail.)
c) Le mail reçu contient un code de réinitialisation. Ce code n'est valide que pour cette carte (adéquation unique entre les deux). De ce fait, et pour des raisons de sécurité, aucun mot de passe ou identifiant ne doit être stocké sur le serveur.
d) Le client copie ce code de réinitialisation sur sa carte. Celle-ci lui demande alors un nouvel identifiant et mot de passe pour sa carte. (Seule la carte stocke ces identifiant et mot de passe également pour des raisons de sécurité)

### 9. Lien espace personnel public hébergé / espace confidentiel médical embarqué

La création d'un espace personnel lors de l'activation de la carte permet de créer 2 univers distincts :
- l'un médical et confidentiel contenant le profil médical confidentiel sécurisé et accessible uniquement sur le support local par un code d'accès ou une clé de substitution via un tiers de confiance. Ces données médicales confidentielles ne sont pas hébergées et sont physiquement totalement maîtrisées par le patient, dont il faut voler la carte avant tout idée de piratage informatique.
   Ces données sont physiquement la propriété de l'utilisateur qui maîtrise son éventuelle exploitation (statistiques, recherche médicale anonyme...)
- L'autre administratif et publique, hébergé sur le serveur dans un espace personnel ne contenant pas de données médicales à l'exception éventuellement du « profil médial publique ».
Cet espace ouvert au monde de l'internet est destiné à permettre un partage de données en lien notamment avec les applications des objets connectés d'aujourd'hui. En outre il peut constituer un espace ouvert à différents services de conseils ou de surveillance pour des données paramédicales moins sensibles (surveillance poids, tension, masse grâce, sommeil...)
Des partages et échanges sur les réseaux sociaux de ces données sont alors possibles tout en gardant une maîtrise des données confidentielles qui restent protégées sur la carte.

### Un lien intéressant peut-être fait entre ces 2 espaces.

- La publication éventuelle dans l'espace hébergé décidée par « consentement patient » du profil médical public.

Mais surtout :
- Le marquage par un bouton similaire à celui de la gestion de confidentialité, au sein de l'applicatif embarqué, sur chaque question qui donnerait la possibilité à l'utilisateur de « taguer » certaines questions médicales afin de recevoir des conseils médicaux liés à la pathologie concernée.
- Ce bouton pourrait être dénommé (Tag Conseil »
- Ces conseils pourraient se matérialiser par des documents PDF ou autres, mais aussi par des vidéos ciblées (exercices et conseils appropriés) dans le domaine médical, sportif, nutrition, physiologie, prévention...
- A la fermeture de l'applicatif, les questions marquées/choisies sont envoyés sur un « serveur conseil » indépendant qui créerait un lien dans l'espace personnel de l'utilisateur avec le document ou la vidéo conseil concernée.
- On peut également décider d'automatiser la marquage lors d'une réponse positive à une pathologie ; exemple - êtes-vous diabétique, si oui le « tag conseil » s'active
- la liberté d'automatisation sera laissée à l'appréciation de l'utilisateur.
- Dans son espace personnel, le patient verrait apparaître le conseil qu'il peut alors consulter, stocker ou effacer.
- Aucun lien direct ne serait fait avec la pathologie concernée mais les propositions de conseil, de suivi ou d'alertes seraient ainsi personnalisés tout en conservant la séparation données publiques/données confidentielles axe prioritaire de notre brevet.

### 10. Avantages de l'invention

La présente invention se propose de combiner sur un même support externe, telle une carte médicale au format « carte de crédit » un ensemble de circuits électroniques et moyens permettant de combiner la fonctionnalité USB permettant une grande flexibilité pour la constitution, la consultation et la mise à jour du dossier médicale avec la fonctionnalité de la carte à puce/RFID qui est à la base du système administratif de facturation tiers-payant (à l'instar du Sesam-Vitale français)

C'est ainsi que la carte médicale décrite précédemment s'avérerait des plus utiles, notamment dans des pays où n'existe pas de système de dématérialisation de feuille de soins pour le tiers payant (public ou privée).

L'intérêt est de séparer de manière distincte et en conformité avec toutes les législations internationales, les données nominatives administratives des données médicales sensibles et confidentielles.

Cette séparation est particulièrement opportune sur le plan pratique car les données administratives ne sont en général pas traitées dans le même temps de la consultation et la saisie de ces informations est souvent distincte, effectuée sur le plan administratif, le plus souvent par la secrétaire alors que le dossier médical est rempli par le médecin.
Le recours à un lecteur sans contact RFID, NFC , voire à un lecteur de carte à puce, reste compatible avec cette gestion purement administrative, tandis que l'utilisation du port USB avec mot de passe pour les données médicales confidentielles ouvre le champ des possibles de la numérisation du dossier par d'autres acteurs de la santé que le médecin (infirmière, physiothérapeute..) et surtout par le patient lui-même qui décide de s'impliquer dans la gestion de son dossier médical.

Par ailleurs, cette séparation est une opportunité intéressante pour la « CNIL » et toutes les législations équivalentes, en séparant clairement les applicatifs et distinguant les modes d'accès aux informations, tout en les réunissant sur un même support externe. La confidentialité, la propriété et l'exploitation de ces données très confidentielles est alors rendue aux patients pour une meilleure sécurisation de l'information qui se trouve ainsi « éclatée » sur chaque support, et non centralisée et hébergée dans des « clouds » rendus attractifs au piratage informatique.

La carte médicale duale apporte une solution du plus grand intérêt pour les patients mais également les praticiens.

### Avantages patients

- Une accessibilité immédiate à ses informations médicales dans un classement adaptée à une bonne lecture médicale
- Une prise de contrôle de la gestion de ses données de santé (protection, propriété, exploitation...)

### Avantages médecins

- Des informations médicales valables accessibles en un clic
- La confidentialité préservée grâce à la création d'un profil médical publique et confidentiel géré par le patient, donc juridiquement incontestable.
- Un gain de temps précieux dans la prise des informations essentielles du patient
- Une présentation numérique et ordonnée des documents médicaux essentiels créant une justification médicale indispensable
- L'indication du nombre d'informations confidentielles et de la date de dernière mise à jour indispensable à la validité du dossier médical

Il s'agit par ailleurs d'une solution particulièrement intéressante d'un point de vue juridique car elle est compatible avec la réglementation applicable aux données personnelles dans la majorité des pays. Elle est « CNIL compatible » quel que soit le pays.

La législation sur l'hébergement et la protection des données personnelles ne s'applique pas dans le cas de ce concept sur support externe de stockage véhiculé par le patient, et ce en conformité avec toutes les législations internationales, car :
- L'utilisateur est le propriétaire de la carte,
- L'utilisateur décide des données qu'il souhaite intégrer sur la carte,
- L'utilisateur décide des données qu'il souhaite rendre visibles ou non sur la carte,
- Il n'y a aucun hébergement nominatif centralisé.
- Il n'existe aucun hébergement centralisé des données de santé. Toutes les informations sont sécurisées et enregistrées dans le logiciel embarqué sur la carte,
- Les seuls liens internet interviennent à l'activation de la carte, à la modification du questionnaire ou à la demande d'un mot de passe égaré. Seul le fichier client est centralisé. Il n'existe aucune donnée de santé hébergée. C'est un point fondamental pour la Commission nationale de l'informatique et des libertés (CNIL),
Par ailleurs, aucun lien n'est possible entre les données médicales de la carte (sur laquelle ne figure ni nom, ni numéro) et le fichier client,
En donnant la main au patient qui gère lui-même l'affichage du profil médical public et/ou confidentiel, on reste strictement dans l'esprit du législateur.

### 11. Conclusion

La présente description a montré l'importance du « Profil médical d'urgence » qui permet de présenter des données médicales essentielles en cas d'urgence selon différents processus d'ouverture d'accès.

Globalement, le procédé technique décrit, permet à la fois la création sécurisée (certification et identification) d'une ouverture d'accès à un praticien, en même temps qu'elle en définit le périmètre de droit d'accès selon le praticien concerné et l'authentification forte du patient comme celle du praticien.
La philosophie du brevet est de mettre le patient au coeur du système d'information concernant le partage de son Dossier Médical Personnel à travers les professionnels de santé médicaux ou para-médicaux, quel que soit l'endroit où il se trouve dans le monde.
L'idée est de donner au patient la possibilité d'ouvrir des droits d'accès à son dossier médical personnel essentiel, à tout praticien n'appartenant à un réseau d'information défini dans un système spécifique (différent selon les pays), que ce soit de façon temporaire ou permanente, pour des informations complètes ou partielles et ce dans toutes circonstances, et notamment dans les situations d'urgence (patient conscient ou inconscient) afin d'améliorer sa sécurité médicale. Le patient, ainsi mis au coeur du système, est en capacité et en droit de définir lui-même les informations qu'il souhaite rendre accessible immédiatement et sans contrainte, de celles qu'il souhaite protéger. Ce « Profil médical confidentiel » est sécurisé par différents processus nécessitant un code confidentiel de « déverrouillage » pour l'ouverture de ces informations dites sensibles.
Le procédé présente une solution technique basée sur un support externe (carte, clé, téléphone...) qui permet l'identification du praticien et du patient, et permet en même temps le déclenchement de l'ouverture du Profil Médical via le web. Le patient est identifié sur support (identifiant généré lors de l'abonnement) - carte, clé ou autre moyen plus mobile (téléphone..)

La présente demande vise à réunir sur un même support au format d'une carte de crédit des systèmes d'exploitation distincts, l'un sous format USB contenant un procédé de génération d'un dossier médical numérique et l'autre sous format puce à contact type RFID ou carte à puce, destiné à authentifier le « couple médecin/patient » d'un point de vue administratif et à gérer les données administratives et financière de remboursement des actes médicaux (type tiers payant ou autre).

Par ailleurs l'extension du processus d'activation de la carte à un abonnement spécifique lié à l'extension de services ou de questionnaires, offre ainsi la possibilité de création d'un espace personnel hébergé pouvant servir à la restitution de mots de passe et permettre de créer des liens entre les données médicales non hébergées avec cet espace personnel et offrir aux usagers des possibilités de conseils médicalement ciblés et personnalisés, tout en conservant une sécurisation importante de ces informations sensibles et confidentielles.

Les procédés qui ont été décrits permettent :
- Activation du programme informatique embarqué sur le support USB permettant la saisie par le patient ou un professionnel de santé d'un dossier Questionnaire médical conçu de manière à permettre la collecte de ses données médicales personnelles, et le stockage de celles-ci dans une base de données disposée au sein dudit support de stockage, la saisie étant associée à l'attribution d'un caractère confidentiel ou non à chacune des informations saisies par le titulaire du dossier médical
- création d'un espace personnel administratif hébergé sur le serveur d'activation et système de mise en place d'un abonnement lié aux différents types de questionnaires médicaux proposés. Processus de régénération de mot de passe en lien avec cet espace personnel hébergé
- création d'un lien par « marquage » de certaines questions sur le programme informatique embarqué sur le port USB afin de permettre un lien à l'enregistrement destiné à la mise en place de conseils personnalisés « anonymes ».
- Authentification médecin et patients par la validation conjointe des deux cartes grâce aux logiciels contenus sur la puce RFID (ou carte à puce selon le processus choisi dans chaque pays) et traitement administratif du tiers payant consécutive à l'authentification conjointe patient/médecin.
- Génération automatisée d'une clé de substitution par l'authentification conjointe médecin/patient vers un serveur externe destinée à l'ouverture du profil médical confidentiel.
- transmission de la requête au dit serveur, qui délivre une clé de substitution et ouvre instantanément l'accès aux données médicales confidentielles, ladite requête permettant un enregistrement administratif auprès dudit serveur et une traçabilité juridique du processus.

## Revendications

1. Support de stockage électronique pour un dossier médical numérique comportant un espace de stockage pour ledit dossier médical numérique, ainsi qu'un programme informatique résidant sur ledit support, ledit support comportant une première zone de stockage sécurisée et une seconde zone de stockage non sécurisée (25), dans lequel ledit support de stockage comporte un port USB (22) ainsi que des premiers circuits (22) configurés pour permettre la création, la consultation et la mise à jour du dossier médical numérique via ledit port USB, dans lequel le support de stockage électronique comporte une puce d'authentification pour le stockage de données administratives afférentes au patient et de second circuits (23, 26) permettant l'authentification du patient via un second canal de communication.

2. Support de stockage électronique pour un dossier médicale dans lequel ledit support se présente sous la forme d'une carte médicale et dans lequel lesdits second circuits comporte un système RFID permettant une authentification forte du patient via une communication sans fil.

3. Support de stockage électronique pour un dossier médical dans lequel ledit support se présente sous la forme d'une carte médicale dans laquelle lesdits seconds circuits comportent une carte à puce permettant une authentification forte du patient.

4. Support de stockage selon l'une des revendications précédentes **caractérisé en ce qu'**il permet la génération d'un Profil Médical d'Urgence généré à partir des informations collectées au sein dudit dossier médical, dans lequel ledit Profil Médical d'urgence est accessible librement via le port USB.

5. Support de stockage selon la revendication 4 dans lequel ledit support est configuré pour autoriser un libre accès au dossier médical complet sur double authentification du patient et du praticien, dans lequel l'authentification du patient est réalisé par la mise en oeuvre d'un programme se déroulant chez un praticien et en présence d'une carte professionnelle dudit praticien et dans lequel l'authentification du praticien est établie par la présence de la carte professionnelle dudit praticien et de la présentation de son mot de passe.

6. Support de stockage selon la revendication 5 dans lequel ledit support est configuré pour autoriser un accès automatique audit dossier médical sur présence d'un logiciel de facturation ayant également authentifié le praticien.

7. Support de stockage selon la revendication 6 dans lequel le programme informatique est configuré pour permettre la saisie par le patient d'un dossier Questionnaire médical conçu de manière à permettre la collecte de ses données médicales personnelles, et le stockage de celles-ci dans une base de données disposée au sein dudit support de stockage, la saisie étant associée à l'attribution d'un caractère confidentiel ou non à chacune des informations saisies par le titulaire du dossier médical;
dans lequel ledit programme informatique est configuré pour générer automatiquement un profil médical personnel d'urgence correspondant au patient, par exemple dans un format électronique pouvant être disponible en plusieurs langues, ne comportant que les seules données auxquelles ont été attribuées un caractère non confidentiel.

8. Support de stockage selon la revendication 7 dans lequel le programme informatique est configuré pour permettre la saisie d'une étiquette (*Tag Conseil*) déterminant un intérêt particulier pour le patient pour un aspect particulier du Questionnaire médical, et dans lequel le programme informatique est configuré pour transmettre un second profil « conseil » à un serveur externe en vue de l'obtention de services supplémentaires dudit serveur externe relativement à l'aspect particulier identifié par ladite étiquette.

9. Support selon l'une quelconque des revendications précédentes, **caractérisé en ce qu'**il comporte des moyens permettant un accès indirect au dossier médical numérique confidentiel et/ou nominatif via un tiers de confiance.

10. Procédé de génération d'un dossier médical numérique destiné à être stocké sur un support amovible, tel qu'une carte à mémoire comportant un premier moyen de communication de type USB et un second moyen de communication sécurisé, tel que filaire (carte à puce) ou non filaire (RFID, NFC), comportant un espace de stockage ainsi qu'un programme informatique résidant sur ledit support,
ledit procédé comportant les étapes:
- branchement dudit support par le port USB à un ordinateur (401);
- création d'un nouveau compte et activation de la carte médicale auprès d'un serveur externe (402) ;
- démarrage (403) dudit programme informatique permettant la saisie par le patient d'un dossier Questionnaire médical conçu de manière à permettre la collecte de ses données médicales personnelles, et le stockage de celles-ci dans une base de données disposée au sein dudit support de stockage, la saisie étant associée à l'attribution d'un caractère confidentiel ou non à chacune des informations saisies par le titulaire du dossier médical ainsi qu'à une étiquette (*Tag Conseil*) déterminant un intérêt particulier pour le patient ;
- génération (407) automatique d'un profil médical personnel d'urgence correspondant au patient ne comportant que les seules données auxquelles ont été attribuées un caractère non confidentiel ;
- génération (408) d'un second profil médical « conseil » à partir des étiquettes saisies par le patient et transmission dudit second profil « conseil » audit serveur externe en vue de l'obtention de services complémentaires dudit serveur externe.

11. Procédé d'accès à un dossier médical numérique stocké sur un support de stockage défini dans l'une des revendications 1 à 8, **caractérisé en ce qu'**il comporte les étapes :
- insertion dudit support dans l'ordinateur (10) d'un praticien consulté par le patient titulaire dudit support ;
- mise en oeuvre d'un code informatique stocké sur ledit support et lecture (501) des données administratives contenues dans ledit support pour procéder à une première authentification d'un patient titulaire dudit support de stockage ;
- lecture d'une carte médicale professionnelle dudit praticien (504) et vérification du mot de passe associé (506) en vue d'une seconde authentification portant sur ledit praticien ;
- en réponse à ladite première et seconde authentification, ouverture du dossier médical numérique (507) pour consultation et/ou mise à jour (510, 511, 512).

12. Procédé d'accès selon la revendication 11 **caractérisé en ce que** le support de stockage est configuré pour une lecture RFID des données administratives du patient.

13. Procédé d'accès selon la revendication 11 **caractérisé en ce que** le support de stockage est configuré pour faire apparaître la photographie de son titulaire et **en ce que** lesdits premiers circuits contenus dans ledit support sont configurés pour permettre l'ouverture du dossier médical numérique uniquement à la suite de la vérification par le praticien de la concordance de la photographie avec ledit patient.

14. Procédé d'accès selon la revendication 11 **caractérisé en ce que** lesdits premiers circuits dudit support de stockage sont configurés pour permettre l'ouverture du dossier médical numérique uniquement à la suite de la vérification que le praticien est bien reconnu par le logiciel de facturation fonctionnant sur ledit ordinateur.

15. Procédé d'accès selon la revendication 11 **caractérisé en ce que** le procédé complète l'ouverture du dossier médical numérique (507) pour consultation et/ou mise à jour par l'édition d'une feuille de soins pouvant être directement transmise à un organisme payeur.
